(19) Europäisches Patentamt
European Patent Office
Office européen des brevets

(11) **EP 4 495 099 A1**

(12) **EUROPEAN PATENT APPLICATION**
published in accordance with Art. 153(4) EPC

(43) Date of publication:
**22.01.2025 Bulletin 2025/04**

(21) Application number: **23770872.2**

(22) Date of filing: **16.03.2023**

(51) International Patent Classification (IPC):
*C07C 63/26* [(2006.01)]     *B01D 53/28* [(2006.01)]
*B01J 20/26* [(2006.01)]     *C07C 63/331* [(2006.01)]
*C07F 3/02* [(2006.01)]      *C07F 5/00* [(2006.01)]
*C07F 5/06* [(2006.01)]

(52) Cooperative Patent Classification (CPC):
**B01D 53/28; B01J 20/26; C07C 63/26;
C07C 63/331; C07F 3/02; C07F 5/00; C07F 5/06**

(86) International application number:
**PCT/JP2023/010265**

(87) International publication number:
**WO 2023/176919 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022 JP 2022042378**

(71) Applicants:
• **Kyoto University
Kyoto-shi, Kyoto 606-8501 (JP)**
• **Sumitomo Chemical Company, Limited
Tokyo 103-6020 (JP)**

(72) Inventors:
• **KITAGAWA, Susumu
Kyoto-shi, Kyoto 606-8501 (JP)**
• **HIGUCHI, Masakazu
Kyoto-shi, Kyoto 606-8501 (JP)**
• **OTAKE, Kenichi
Kyoto-shi, Kyoto 606-8501 (JP)**
• **KIKUCHI, Yuta
Ichihara-shi, Chiba 299-0195 (JP)**

(74) Representative: **Vossius & Partner
Patentanwälte Rechtsanwälte mbB
Siebertstrasse 3
81675 München (DE)**

(54) **METAL-ORGANIC FRAMEWORK**

(57) An object of the present invention is to provide a MOF having an excellent heat resistance. A metal organic framework, comprising: an organic ligand; a structure $S_x$ constituted by one or more of $O^{2-}$, $OH_2$, $OH^-$, $OCH_3^-$, and $OC_2H_5^-$; and a metal ion, wherein the metal organic framework has a structure $S_{M-x}$, in the structure $S_{M-x}$, two or more metal ions are bonded to one oxygen atom in the structure $S_x$, the number of the metal ions in a unit lattice is two or more, and a water absorption rate A obtained by formula (1) is 25% or more.

$$\text{water absorption rate } A = (W_1 - W_2)/W_2 \qquad (1)$$

(in the formula, $W_1$ represents a mass of the metal organic framework after being left until an equilibrium moisture content is reached under conditions of a temperature of 25°C and a relative humidity of 50%, and $W_2$ represents a mass of the organic metal framework after obtaining the mass $W_1$ and being left at a temperature of 200°C for 1 hour)

EP 4 495 099 A1

**Description**

TECHNICAL FIELD

The present invention relates to a metal organic framework.

BACKGROUND ART

Metal organic frameworks are also referred to as porous coordination polymers and are a class of materials which form porous structures by coordinate bonds between metal ions and organic ligands, and are expected to adsorb and desorb gas and expected to be applied to catalysts, and the like.

For example, Patent Literature 1 discloses a metal organic framework including metal ions, first ligands, second ligands, and optional third ligands. In the metal organic framework, the metal ion is an aluminum ion; the first and second ligands are each an ion of an organic compound that includes a hetero ring with two carboxyl groups; a heteroatom and the angle formed between the carboxyl groups satisfy a predetermined condition; the third ligand is an ion of an organic compound with two carboxyl groups; and the existing proportion of each of the first to third ligands is within a predetermined range.

CITATION LIST

PATENT LITERATURE

Patent Literature 1: Japanese Laid-Open Patent Publication No.2020-176101

SUMMARY OF THE INVENTION

PROBLEMS TO BE SOLVED BY THE INVENTION

The metal organic framework (hereinafter, may be referred to as MOF) is used in a high-temperature environment in some cases and preferably has excellent heat resistance. However, previously proposed MOFs still needs improvement in heat resistance.

Therefore, an object of the present invention is to provide a MOF having an excellent heat resistance.

SOLUTION TO THE PROBLEMS

The present invention that can achieve the problem is as follows.

[1] A metal organic framework, comprising:

an organic ligand;
a structure $S_x$ constituted by one or more of $O^{2-}$, $OH_2$, $OH^-$, $OCH_3^-$, and $OC_2H_5^-$; and
a metal ion, wherein
the metal organic framework has a structure $S_{M-x}$,
in the structure $S_{M-x}$, two or more metal ions are bonded to one oxygen atom in the structure $S_x$,
the number of the metal ions in a unit lattice is two or more, and
a water absorption rate A obtained by formula (1) is 25% or more.

$$\text{water absorption rate } A = (W_1 - W_2)/W_2 \qquad (1)$$

(in the formula, $W_1$ represents a mass of the metal organic framework after being left until an equilibrium moisture content is reached under conditions of a temperature of 25°C and a relative humidity of 50%, and $W_2$ represents a mass of the organic metal framework after obtaining the mass $W_1$ and being left at a temperature of 200°C for 1 hour)

[2] The metal organic framework according to [1], wherein

the metal organic framework has a cluster structure constituted by the structure $S_x$ and the metal ions,

in the cluster structure, the metal ions are bonded to each other via the oxygen atom of the structure $S_x$, and the number of the metal ions in one cluster structure is three or four.

[3] The metal organic framework according to [1] or [2], wherein
the organic ligand includes at least one selected from the group consisting of carboxylates represented by $R(COO^-)_n$ (R represents an n-valent group, and n represents an integer of 2 or more) and an oxalate ion $(COO^-)_2$.
[4] The metal organic framework according to any one of [1] to [3], wherein
the structure $S_x$ is constituted by one or more selected from $O^{2-}$, $OH_2$, $OH^-$, and $OCH_3^-$.
[5] The metal organic framework according to any one of [1] to [4], wherein
the metal ion is an ion of at least one metal selected from elements in groups 2 to 14 in the third to sixth periods in a periodic table.

ADVANTAGEOUS EFFECTS OF THE INVENTION

According to the present invention, it is possible to provide MOF that exhibits an excellent heat resistance.

DESCRIPTION OF EMBODIMENTS

The present invention is directed to a MOF comprising an organic ligand, a structure $S_x$ constituted by one or more of $O^{2-}$, $OH_2$, $OH^-$, $OCH_3^-$, and $OC_2H_5^-$, and a metal ion and has a structure $S_{M-x}$ in which two or more metal ions are bonded to one oxygen atom in the structure $S_x$. In the MOF, the number of the metal ions in a unit lattice is two or more, and a water absorption rate A obtained by formula (1) is 25% or more.

$$\text{water absorption rate } A = (W_1 - W_2)/W_2 \qquad (1)$$

(in the formula, $W_1$ represents the mass of the metal organic framework after being left until the equilibrium moisture content is reached under the conditions of a temperature of 25°C and a relative humidity of 50%, and $W_2$ represents the mass of the organic metal framework after obtaining the mass $W_1$ and being left at a temperature of 200°C for 1 hour)
The structure $S_x$ can be coordinated to two or more metal ions at an oxygen atom moiety, and can act as a ligand. In addition, to $O^{2-}$, $OH_2$, $OH^-$, $OCH_3^-$, and $OC_2H_5^-$, only metal ions are bonded at an oxygen atom, and another group is not bonded. It is considered that the structure $S_{M-x}$ in which two or more metal ions are bonded to one oxygen atom in the structure $S_x$ is included, in other words, a structure in which the metal ions are collected through the oxygen atom of the structure $S_x$ is included, and thus heat resistance to be evaluated through a decomposition start temperature is improved. In addition, a high water absorption rate, that is, high resistance to water, means that a MOF structure itself is strong. Therefore, it is considered that the high water absorption rate also contributes to improvement in water resistance to be evaluated through a decomposition start temperature.
The number of the metal ions in the unit lattice being two or more means that the number of the metal ions in the minimum repeating unit of a repeating structure of a MOF, for example, the minimum repeating unit (unit lattice) in repeating of the bond between the structure $S_x$ and metal, is two or more. Therefore, when the MOF, for example, has a so-called chain-type structure in which the linear bond between the structure $S_x$ and the metal is repeated in this order multiple times (e.g., structure $S_x$-metal-structure $S_x$-metal-structure $S_x$-metal, or the like), the structure $S_x$-metal can be considered as the minimum repeating unit, and the number of the metal ions bonded to the structure $S_x$ is one. Thus, such a MOF having the chain-type structure is not generally included in the present invention.
The structure $S_x$ is constituted by preferably at least one selected from $O^{2-}$, $OH_2$, $OH^-$, and $OCH_3^-$, more preferably at least one selected from $O^{2-}$, $OH_2$, and $OH^-$, and further preferably two selected from $O^{2-}$, $OH_2$, and $OH^-$. The MOF of the present invention preferably has a plurality of the structures $S_{M-x}$, and the structures $S_x$ in the plurality of structures $S_{M-x}$ may be the same or different from each other.
The number of the metal ions bonded to one oxygen atom in the structure $S_x$ is two or more and is usually four or less, and the kinds of the metal ions bonded to one oxygen atom in the structure $S_x$ may be the same or different from each other.
The MOF of the present invention preferably has a cluster structure, and the cluster structure means a structure which is constituted by the metal ions and the structure $S_x$, and in which the metal ions are bonded to each other via the structure $S_x$. The MOF is a framework including the metal ions and the organic ligands (however, not including either $OCH_3^-$ or $OC_2H_5^-$, which is not bonded to any group other than metal ions), and when the MOF of the present invention has the cluster structures, the cluster structures are bonded via the organic ligands.
When the MOF has the cluster structures, the total number of $O^{2-}$, $OH_2$, and $OH^-$ which are the structures $S_x$, in one cluster structure is preferably 2 or more and 10 or less, and more preferably 5 or more and 8 or less. When the total

number of $O^{2-}$, $OH_2$, and $OH^-$, which are the structures $S_x$, is in the above range, the cluster structure tends to be formed and the basic skeleton of the MOF is stabilized. As a result, the heat resistance of the MOF is improved.

The BET specific surface area of the MOF is preferably 495 $cm^2/g$ or more, more preferably 1000 $cm^2/g$ or more, and further preferably 1200 $cm^2/g$ or more. The upper limit of the BET specific surface area is not particularly limited, and the BET specific surface area is, for example, 2000 $cm^2/g$ or less.

When the MOF has the cluster structures, the number of the metal ions in one cluster structure is preferably 2 or more, and more preferably 3 or more, and the number of the metal ions in one cluster structure is preferably 10 or less, and more preferably 8 or less. The number of the metal ions in one cluster structure is particularly preferably 3 or 4, and in this case, heat resistance is particularly improved. In the above-described chain-type structure, the number of the metal ions in the cluster structure is counted as 1.

As described above, as for the MOF of the present invention, the water absorption rate A obtained by the above formula (1) is 25% or more. The water absorption rate A is preferably 27% or more. The upper limit thereof is not particularly limited, and the water absorption rate A may be, for example, 45% or less, 40% or less, or 37% or less.

In the measurement of $W_1$ in the above formula (1), when the metal organic framework is left until the equilibrium moisture content is reached, the metal organic framework may be left in an air atmosphere at a temperature of 25°C and a relative humidity of 50% for 12 hours, as in Examples described below. In the measurement of $W_2$, a heating rate at the time of heating the organic metal frame work, for which the mass $W_1$ has been obtained, to 200°C is preferably 5°C/min.

A metal ion constituting the MOF is preferably an ion of at least one metal selected from elements in groups 2 to 14 in the third to sixth periods in the periodic table. In the present specification, the term "metal" is used to include metalloids such as Si and Ge. The metal ion constituting the MOF is preferably an ion of at least one metal selected from the group consisting of Mg, Al, Ga, In, Ti, V, Cr, Mn, Fe, Co, Ni, Cu, Zr, and Hf, and is more preferably an ion of at least one metal selected from the group consisting of Al, Ti, Cu, In, and Mg, and further preferably an ion of at least one metal selected from the group consisting of Al, In, and Mg.

An organic ligand constituting the MOF preferably includes at least one selected from the group consisting of carboxylates (carboxylato) represented by $R(COO^-)_n$ (R represents an n-valent group, and n represents an integer of 2 or more) and an oxalate ion $(COO^-)_2$. R preferably represents an aliphatic chain hydrocarbon group, an aliphatic cyclic hydrocarbon group, an aliphatic heterocyclic hydrocarbon group (group in which one or more carbon atoms of an aliphatic cyclic hydrocarbon group are substituted with heteroatoms), an aromatic hydrocarbon group, or an aromatic heterocyclic hydrocarbon group (group in which one or more carbon atoms of an aromatic hydrocarbon group are substituted with heteroatoms). The aliphatic chain hydrocarbon group may be a linear chain structure or a branched chain structure, and may be a saturated hydrocarbon group or an unsaturated hydrocarbon group. A heteroatom in the aliphatic heterocyclic hydrocarbon group or the aromatic heterocyclic hydrocarbon group is preferably nitrogen.

n is 2 or more, preferably 4 or less, more preferably 2 or more and 3 or less, and most preferably 2.

The above-described aliphatic chain hydrocarbon group, aliphatic cyclic hydrocarbon group, aliphatic heterocyclic hydrocarbon group, aromatic hydrocarbon group, and aromatic heterocyclic hydrocarbon group further include one or more functional groups X selected from the group consisting of a carboxylic acid anhydride group, -OH, -OR$^1$, -NH$_2$, -NHR$^1$, -N(R$^1$)$_2$, -CN, a halogeno group, -C(=S)SH, -C(=O)SH and a tautomer thereof, and -SO$_3$H. Each R$^1$ represents an alkyl group having 1 or 2 carbon atoms. As the above functional group X, -OH or -NH$_2$ is particularly preferable.

Examples of the aromatic heterocyclic hydrocarbon group include pyrazole, imidazole, thiazole, oxazole, pyridine, pyrimidine, pyridazine, pyrazine, and triazine, and pyrazole is particularly preferable.

R preferably represents at least one of an unsaturated linear hydrocarbon group, an aromatic hydrocarbon group, and a hydrocarbon group containing an aromatic ring that includes a nitrogen atom, which may have the above-described functional group X, and an aromatic hydrocarbon group that doesn't have the functional group X is more preferable.

The number of carbon atoms of R is preferably 1 or more, and more preferably 2 or more, and is preferably 30 or less. The number of carbon atoms or R is further preferred in the order of 6 or more, 10 or more, 15 or more, and 20 or more, and the upper limit thereof may be 28 or less, 24 or less, 18 or less, or 10 or less.

In particular, the organic ligand constituting the MOF preferably includes at least one selected from the group consisting of carboxylates represented by $R(COO^-)_2$ and an oxalate ion $(COO^-)_2$, and, specifically, examples thereof include a ligand in which two protons are removed from two carboxyl groups (-COOH) of a dicarboxylic acid, and a ligand in which three protons are removed from three carboxyl groups of a tricarboxylic acid.

Examples of the dicarboxylic acid include oxalic acid, succinic acid, fumaric acid, tartaric acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8- heptadecanedicarboxylic acid, 1,9-heptadecanedicarboxylic acid, heptadecanedicarboxylic acid, acetylenedicarboxylic acid, 1,2-benzene dicarboxylic acid (phthalic acid), 1,3-benzene dicarboxylic acid (isophthalic acid), 2,3-pyridine dicarboxylic acid, 1,3-butadiene-1,4-dicarboxylic acid, 1,4-benzene dicarboxylic acid (terephthalic acid), 2-aminoterephthalic acid, 2,5-dihydroxyterephthalic acid, imidazole-2,4-dicarboxylic acid, 3,5-pyrazole dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminodiphenylmethane-3,3'-dicarboxylic acid, quinoline-3,4-dicarboxylic acid, 7-chloro-4-hydroxyquinoline-2,8-dicarboxylic acid, diimide dicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylene dicarboxylic acid, Pluriol E 200-dicarboxylic acid, 3,6-dioxaoctane dicarboxylic acid, 3,5-cyclohexadiene-1,2-dicarboxylic acid, octane dicarboxylic acid, pentane-3,3-carboxylic acid, 4,4'-diamino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyl dicarboxylic acid, 7-chloro-8-methylquinoline-2,3-dicarboxylic acid, 1-anilino anthraquinone-2,4'-dicarboxylic acid, polytetrahydrofuran 250-dicarboxylic acid, 1,4-bis(carboxymethyl) piperazine-2,3-dicarboxylic acid, 7-chloroquinoline-3,8-dicarboxylic acid, 1-(4-carboxy)phenyl-3-(4-chloro) phenylpyrazoline-4,5-dicarboxylic acid, 1,4,5,6,7,7-hexachloro-5-norbornene-2,3-dicarboxylic acid, phenylindan dicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidine-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenone dicarboxylic acid, Pluriol E 300-dicarboxylic acid, Pluriol E 400-dicarboxylic acid, Pluriol E 600-dicarboxylic acid, pyrazole-3,4-dicarboxylic acid, 2,3-pyrazine dicarboxylic acid, 5,6-dimethyl-2,3-pyrazine dicarboxylic acid, bis(4-aminophenyl)ether diimide-dicarboxylic acid, 4,4'-diaminodiphenylmethane diimide-dicarboxylic acid, bis(4-aminophenyl)sulfone diimide-dicarboxylic acid, 1,4-naphthalene dicarboxylic acid, 2,6-naphthalene dicarboxylic acid, 1,3-adamantane dicarboxylic acid, 1,8-naphthalene dicarboxylic acid, 2,3-naphthalene dicarboxylic acid, 8-methoxy-2,3-naphthalene dicarboxylic acid, 8-nitro-2,3-naphthalene dicarboxylic acid, 8-sulfo-2,3-naphthalene dicarboxylic acid, anthracene-2,3-dicarboxylic acid, 2',3'-diphenyl-p-terphenyl-4,4"-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzene dicarboxylic acid, 7,8-quinoline dicarboxylic acid, 4,5-imidazole dicarboxylic acid, 4-cyclohexene-1,2-dicarboxylic acid, hexatriacontane dicarboxylic acid, tetradecanedicarboxylic acid, 1,7-heptanedicarboxylic acid, 5-hydroxy-1,3-benzene dicarboxylic acid, 2,5-dihydroxy-1,4-benzene dicarboxylic acid, pyrazine-2,3-dicarboxylic acid, furan-2,5-dicarboxylic acid, 1-nonene-6,9-dicarboxylic acid, eicosene dicarboxylic acid, 4,4'-dihydroxydiphenylmethane-3,3'-dicarboxylic acid, 1-amino-4-methyl-9,10-dioxo-9,10-dihydroanthracene-2,3-dicarboxylic acid, 2,5-pyridine dicarboxylic acid, cyclohexene-2,3-dicarboxylic acid, 2,9-dichlorofluorubine-4,11-dicarboxylic acid, 7-chloro-3-methylquinoline-6,8-dicarboxylic acid, 2,4-dichlorobenzophenone-2',5'-dicarboxylic acid, 1,3-benzene dicarboxylic acid, 2,6-pyridine dicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazole dicarboxylic acid, 2-nitrobenzene-1,4-dicarboxylic acid, heptane-1,7-dicarboxylic acid, cyclobutane-1,1-dicarboxylic acid, 1,14-tetradecanedicarboxylic acid, 5,6-dehydronorbornane-2,3-dicarboxylic acid, 5-ethyl-2,3-pyridine dicarboxylic acid, and camphordicarboxylic acid. Terephthalic acid is most preferred.

Examples of the tricarboxylic acid include tricarballylic acid, aconitic acid, trimellitic acid, trimesic acid, biphenyl-3,4',5-tricarboxylic acid, and 1,3,5-tris(4-carboxyphenyl)benzene, and 1,3,5-tris(4-carboxyphenyl)benzene is preferable.

As the organic ligand constituting the MOF of the present invention, a different type of an organic ligand other than carboxylates represented by $R(COO^-)_n$ and an oxalate ion $(COO^-)_2$ may be further included, and examples of the organic ligand include at least one selected from the group consisting of urea, pyrazine, oxazole, isoxazole, thiazole, imidazole, pyrazole, 1,2,3 -thiadiazole, pyridazine, pyrimidine, purine, and pteridine.

The molar ratio of the metal ion to the organic ligand (total amount thereof, in a case of a plurality of kinds of organic ligands) (metal ion/organic ligand) is preferably 0.5 or more, more preferably 1.0 or more, further preferably 2.0 or more, and the molar ratio is preferably 5 or less, more preferably 4 or less, and further preferably 3 or less.

Next, a method for producing the MOF of the present invention will be described. The MOF of the present invention can be obtained by reacting, in a solvent, a metal compound containing the metal ion constituting the MOF and one or more kinds of organic compounds for the organic ligands constituting the MOF.

The metal compound that contains the metal ion constituting the MOF is preferably metal sulfate, metal acetate, metal nitrate, metal chloride, or metal alkoxide, is more preferably metal nitrate, or metal chloride, and is further preferably metal nitrate.

The organic compound for the organic ligands constituting the MOF is preferably one or more selected from the group consisting of polyvalent carboxylic acids represented by $R(COOH)_n$ and oxalic acid. R and n are equivalent to R and n described above for the carboxylates represented by $R(COO^-)_n$, and all the above descriptions about R and n, also including preferable aspects thereof, can be referred to.

The organic compound preferably includes at least one selected from the group consisting of tricarboxylic acid and dicarboxylic acid. For specific examples thereof, the tricarboxylic acid and the dicarboxylic acids described for the carboxylates represented by $R(COO^-)_n$ and preferable ranges thereof can be referred to.

As the organic compound for the organic ligands, in addition to one or more selected from the group consisting of the polyvalent carboxylic acids represented by $R(COOH)_n$ and oxalic acid, at least one selected from the group consisting of urea, pyrazine, oxazole, isoxazole, thiazole, imidazole, pyrazole, 1,2,3-thiadiazole, pyridazine, pyrimidine, purine, and pteridine is preferably used.

The solvent is not particularly limited as long as the solvent is capable of dissolving the metal compound and the organic compound for the organic ligand, and an appropriate solvent can be selected from water and an organic solvent. The solvent is preferably water, an alcohol-based solvent such as methanol, or an amide-based solvent such as dimethylformamide or dimethylacetamide, and at least the amide-based solvent is more preferably included. The structure $S_x$ of the MOF of the present invention is considered to be derived from such a solvent, or water in the air during the production process.

The ratio of the metal compound to the solvent (metal compound/solvent) is preferably 0.01 mol/L or more, more preferably 0.02 mol/L or more, and further preferably 0.05 mol/L or more, and the ratio is preferably 1 mol/L or less, more preferably 0.5 mol/L or less, and further preferably 0.2 mol/L or less.

The ratio of the organic compound for the organic ligand to the solvent (organic compound/solvent) is preferably 0.005 mol/L or more, more preferably 0.01 mol/L or more, and further preferably 0.02 mol/L or more, and the ratio is preferably 3 mol/L or less, more preferably 1 mol/L or less, and further preferably 0.1 mol/L or less.

The molar ratio between the metal compound and the organic compound may be adjusted such that the ratio of the molar quantity of metal atoms in the metal compound to the molar quantity of the organic compound is equal to the above-described molar ratio of the metal ion to the organic ligand in the MOF.

In order to obtain the MOF of the present invention, it is important to appropriately adjust, particularly, at least any of: (i) a condition for mixing the metal compound and the organic compound for the organic ligand; (ii) the temperature at which the mixed compounds are stirred or left as they are, after the metal compound and the organic compound for the organic ligand have been mixed; and (iii) a post-treatment condition for the reaction product of the metal compound and the organic compound for the organic ligand.

Regarding (i), in mixing of the metal compound and the organic compound for the organic ligand, it is important that the metal compound and the organic compound are completely dissolved. Specifically, in the case where the metal compound and the organic compound are mixed in one go, it is preferable that, after the mixing, stirring treatment (for example, ultrasonic treatment) is, for example, performed for 2 minutes or more to sufficiently stir the solution. In addition, it is also preferable that one of the metal compound and the organic compound is completely dissolved in the solvent first and the other is added thereto, and then, the resulting product is stirred for 5 minutes or more.

Regarding (ii), the stirring temperature after mixing the metal compound and the organic compound for the organic ligand may be room temperature or higher, and thereafter, the mixed compounds are preferably left as they are at 100°C to 200°C for approximately 10 hours to 6 days.

Regarding (iii), in the post-treatment condition for the reaction product of the metal compound and the organic compound for the organic ligand, the reaction product is preferably washed three times or more with the solvent used during the reaction of the metal compound and the organic compound. At this time, the solvent preferably includes, particularly, an amide-based solvent such as dimethylformamide or dimethylacetamide.

The MOF of the present invention can be suitably used to adsorb and remove gas or organic molecules, for example. Examples of the gas include carbon dioxide, hydrogen, carbon monoxide, oxygen, nitrogen, hydrocarbon having 1 to 4 carbon atoms, noble gas, hydrogen sulfide, ammonia, sulfur oxide, nitrogen oxide, and siloxane. Examples of the organic molecule include hydrocarbon having 5 to 8 carbon atoms, alcohol having 1 to 8 carbon atoms, aldehyde having 1 to 8 carbon atoms, carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. The organic molecule may contain an aromatic ring.

This application claims the benefit of priority based on the Japanese Patent Application No. 2022-042378 filed on March 17, 2022. The entire contents of the Japanese Patent Application No. 2022-042378 filed on March 17, 2022, are incorporated herein by reference.

EXAMPLES

The present invention will be described in more detail below by means of examples. The present invention. The present invention is not limited by the following examples, and can also be carried out with appropriate modifications being made within the scope of the gist described above and below, and any of these modifications are included in the technical scope of the present invention.

Example 1

In a 50 mL pressure resistant container (including a Teflon (registered trademark) inner cylinder) made of SUS-304, 0.348 mmol of $In(NO_3)_3 \cdot 5H_2O$, 0.955 mmol of $Mg(NO_3)_2 \cdot 6H_2O$, 0.518 mmol of 1,3,5-tris(4-carboxyphenyl) benzene, 2.5 ml of water, and 13.5 ml of dimethylacetamide were mixed while being stirred at room temperature, and ultrasonic waves was applied to the mixture for 3 minutes to dissolve. Then, the resulting product was left as it was at 100°C for 5 days. The obtained precipitated solid product was subjected to three times of washing with 10 mL of dimethylacetamide and filtering, and the obtained filter cake was dried for 3 days at room temperature, to obtain 0.27 g of a product (yield: 99%).

Example 2

In a 100 mL pressure resistant container (including a Teflon (registered trademark) inner cylinder) made of SUS-304, 5.1 mmol of $AlCl_3$, 2.089 mmol of terephthalic acid, and 60 ml of dimethylformamide were all mixed, and ultrasonic waves was applied to the mixture at room temperature for 5 minutes to completely dissolve. Then, the resulting product was left as it was at 110°C for 20 hours. The obtained precipitated solid product was subjected to three times of washing with 30 mL of dimethylformamide and filtering, and the obtained filter cake was dried under reduced pressure in a vacuum drying oven at 60°C for 30 minutes and at 150°C for 12 hours, to obtain 0.42 g of a product (yield: 99%).

Comparative Example 1

In a 100 mL pressure-resistant container (including a Teflon (registered trademark) inner cylinder) made of SUS-304, 0.5 mmol of terephthalic acid, 3.691 mmol of $Al(NO_3)_3 \cdot 9H_2O$, and 40.2 ml of methanol were mixed, 1.8 ml of a 2 M solution of NaOH in methanol was added thereinto at room temperature, and the resulting mixture was mixed at 25°C. Then, the resulting mixture was left as it was at 125°C for 20 hours. The obtained precipitated solid product was subjected to three times of washing with 30 ml of methanol and filtering, and the obtained filter cake was dried at room temperature for 72 hours, to obtain 0.17 g of a product (yield: 99%).

Comparative Example 2

In a 20 ml eggplant flask, 5.129 mmol of $AlCl_3 \cdot 6H_2O$, 5.2 mmol of NaOH, and 10 ml of ion-exchanged water were mixed, and 5.136 mmol of 2,5-furandicarboxylic acid was added thereinto. Then, the resulting product was refluxed at 100°C for 24 hours, to obtain a suspension. The obtained precipitated solid product was separated by centrifugation and washed three times with 10 ml of water, and the obtained cake was dried in a vacuum drying oven at 30°C for 24 hours, to obtain 1.19 g of a product (yield: 90%).

Comparative Example 3

In a 50 mL pressure resistant container (including a Teflon (registered trademark) inner cylinder) made of SUS-304, 5.468 mmol of $Al(NO_3)_3 \cdot 9H_2O$ and 23 ml of ion-exchanged water were mixed, 5.396 mmol of terephthalic acid was further added thereinto, and the resulting product was left as it was at 150°C for 5 hours. The obtained precipitated solid product was subjected to three times of washing with 30 ml of water and filtering, and the obtained filter cake was dried at 80°C for 24 hours and at 130°C for 24 hours, to obtain 0.83 g of a product (yield: 73.7%).

The products obtained in Examples and Comparative Examples were evaluated by the following methods.

(1) Measurement of water absorption rate

Each of the products (MOFs) obtained in Examples and Comparative Examples was left at a temperature of 25°C for 12 hours under an air atmosphere in which the humidity was adjusted so that the relative humidity was 50%, and then a mass $W_1$ was measured. Thereafter, the MOF for which the mass $W_1$ was measured was heated at a heating rate of 5°C/min. while nitrogen was caused to flow, and was left at a temperature of 200°C for 1 hour, and then, a mass $W_2$ was measured. The value obtained by the following formula (1) was set to a water absorption rate A.

$$\text{water absorption rate A} = (W_1 - W_2)/W_2 \qquad (1)$$

(2) Evaluation of MOF structure

Under the following condition, XRD (X-ray diffraction) measurement was performed to obtain a crystallographic information file (cif), the cif was loaded into software named Mercury (The Cambridge Crystallographic Data Centre) to obtain an image, and the image was used to calculate the numbers of $O^{2-}$, $OH_2$, and $OH^-$ in one cluster structure, the number of the metal ions in one cluster structure, and the number of the metal ions bonded to the oxygen atom of each structure $S_x$.

Device: SmartLab manufactured by Rigaku Corporation
Ray source: Cu
Measurement range: 20=3 to 40°
Step size: 0.01°
Scan speed: 3°/min
Measurement temperature: room temperature (25°C)

(3) Measurement of decomposition start temperature

Each of the obtained MOFs was held at 25°C for 12 hours under an air atmosphere in which the relative humidity was adjusted to 50% to perform pre-treatment. Then, the temperature was increased to 500°C at a heating rate of 5°C/min. while nitrogen was caused to flow. According to DTA measurement defined in General rules for thermal analysis of JIS K0129, a point at which decomposition behavior was observed in this section (25 to 500°C) was set as a decomposition start temperature.

The results are shown in Table 1. Each organic ligand in Table 1 is indicated by the name of carboxylic acid in a state before protons are removed from carboxyl groups. In addition, each nuclei number of cluster structure in Table 1 indicates the number of the metal ions in one cluster structure.

(4) Measurement of BET specific surface area

Since the adsorption occupation area of nitrogen molecules is known in advance, the amount of gas molecules adsorbed on only the surface of the sample was measured, and the surface area of the sample was measured according to a BET adsorption isotherm.

Pre-treatment condition: according to the measurement results from TG-DTA described above, the temperature at which water contained in the sample was able to be removed was checked, and heating under reduced pressure was performed overnight.

Device: BELSORP-mini manufactured by MicrotracBEL
Pre-treatment condition:

(1) A glass bar (for a standard sample tube) for reducing the volume was set in a standard sample tube, and the standard sample tube was sealed with a quick seal. The above sample-tube sets, the number of which corresponded to the number of samples to be measured (a maximum of 3 samples per measurement), were prepared and connected to a pre-treatment device (BELPREP VACII). The air inside the sample tube was discharged, and then $N_2$ gas (having a purity of 99.999% or more) was introduced therein until the pressure reached atmospheric pressure;

(2) Thereafter, the sample tube was removed from the pre-treatment device, and the weight was measured by using a precision balance (that displays four or more decimal places) three times to obtain the average value (W1). When the precision balance was used, an ionizer was used to eliminate the effect of static electricity;

(3) About 50 mg of the sample to be measured was weighed on drug packaging paper, and the sample was directly placed into a spherical part in the lower part of the standard sample tube by using a long stem funnel;

(4) The glass bar was returned into the sample tube, the sample tube was sealed with a quick seal, and then the total weight was measured once and the amount of the added sample was tentatively checked;

(5) The sample tube containing the sample was connected to the pre-treatment device to exhaust gas from the sample tube; and

(6) After the pressure in the sample tube became sufficiently low, heating was started (while vacuuming was continued).

Measurement condition:

(1) After the pre-treatment (vacuum heating) was ended, heat was dissipated from the sample tube while the sample tube was held under reduced pressure. After the temperature reached room temperature, $N_2$ gas was introduced therein until the pressure reached atmospheric pressure. The sample tube was removed from the device;

(2) After the pre-treatment, the weight of the sample tube containing the sample was measured three times by using a precision balance to obtain an average value (W2). The weight of the sample was obtained by performing calculation of W2-W1; and

(3) The weight of the sample, and information on $N_2$ gas at a liquid nitrogen temperature (second virial coefficient, and the like) were inputted to the measurement software, the measurement relative pressure at which measurement was desired to be performed was inputted, and a measurement start button was pressed. Then, according to instructions of the software, a Dewar vessel filled with liquid nitrogen or a sample tube was set, and measurement was performed.

[Table 1]

| | Metal ion | Organic ligand | Metal ion/Organic ligand molar ratio | Water adsorption rate A (wt%) | Number of structure Sx in one cluster structure* | | | | | BET specific surface area (cm$^2$/g) | Cluster structure | Decomposition start temperature (°C) |
| --- | --- | --- | --- | --- | --- | --- | --- | --- | --- | --- | --- | --- |
| | | | | | $O^{2-}$ | $OH_2$ | $OH^-$ | $OCH_3$ | Total | | | |
| Example 1 | In,Mg | 1,3,5-tris(4-carboxyphenyl) benzene | 2.5 | 34.7 | 0 | 4 (2) | 2 (2) | 0 | 6 | 1251 | 4 nuclei (In:2 nuclei, Mg:2 nuclei) | 400 |
| Example 2 | Al | Terephthalic acid | 2.4 | 27.8 | 0 | 0 | 2 (2) | 0 | 2 | 1259 | 3 nuclei | 280 |
| Comparative example 1 | Al | Terephthalic acid | 7.4 | 21.2 | 0 | 0 | 0 | 4 (1) | 4 | 491 | Chain type | 250 |
| Comparative example 2 | Al | 2,5-furandicarboxylic acid | 1.0 | 18.7 | 0 | 0 | 1 (1) | 0 | 1 | 57 | Chain type | 220 |
| Comparative example 3 | Al | Terephthalic acid | 1.0 | 1.1 | 0 | 0 | 1 (1) | 0 | 1 | 22 | Chain type | 220 |

The number in parentheses indicates the number of metal ions bonded to the oxygen atom of each structure Sx.

**Claims**

1. A metal organic framework, comprising:

   an organic ligand;
   a structure $S_x$ constituted by one or more of $O^{2-}$, $OH_2$, $OH^-$, $OCH_3^-$, and $OC_2H_5^-$; and
   a metal ion, wherein
   the metal organic framework has a structure $S_{M-x}$,
   in the structure $S_{M-x}$, two or more metal ions are bonded to one oxygen atom in the structure $S_x$,
   the number of the metal ions in a unit lattice is two or more, and
   a water absorption rate A obtained by formula (1) is 25% or more.

$$\text{water absorption rate } A = (W_1 - W_2)/W_2 \qquad (1)$$

   (in the formula, $W_1$ represents a mass of the metal organic framework after being left until an equilibrium moisture content is reached under conditions of a temperature of 25°C and a relative humidity of 50%, and $W_2$ represents a mass of the organic metal framework after obtaining the mass $W_1$ and being left at a temperature of 200°C for 1 hour)

2. The metal organic framework according to claim 1, wherein

   the metal organic framework has a cluster structure constituted by the structure $S_x$ and the metal ions,
   in the cluster structure, the metal ions are bonded to each other via the oxygen atom of the structure $S_x$, and
   the number of the metal ions in one cluster structure is three or four.

3. The metal organic framework according to claim 1 or 2, wherein
   the organic ligand includes at least one selected from the group consisting of carboxylates represented by $R(COO^-)_n$ (R represents an n-valent group, and n represents an integer of 2 or more) and an oxalate ion $(COO^-)_2$.

4. The metal organic framework according to claim 1 or 2, wherein
   the structure $S_x$ is constituted by one or more selected from $O^{2-}$, $OH_2$, $OH^-$, and $OCH_3^-$.

5. The metal organic framework according to claim 1 or 2, wherein
   the metal ion is an ion of at least one metal selected from elements in groups 2 to 14 in the third to sixth periods in a periodic table.

## INTERNATIONAL SEARCH REPORT

| International application No. |
| --- |
| **PCT/JP2023/010265** |

**A.    CLASSIFICATION OF SUBJECT MATTER**

*C07C 63/26*(2006.01)i; *B01D 53/28*(2006.01)i; *B01J 20/26*(2006.01)i; *C07C 63/331*(2006.01)i; *C07F 3/02*(2006.01)i; *C07F 5/00*(2006.01)i; *C07F 5/06*(2006.01)i
  FI:   C07C63/26 Z CSP; C07C63/331; C07F3/02 Z; C07F5/00 J; C07F5/06 D; B01J20/26 A; B01D53/28

According to International Patent Classification (IPC) or to both national classification and IPC

**B.    FIELDS SEARCHED**

Minimum documentation searched (classification system followed by classification symbols)

    C07C63/26; B01D53/28; B01J20/26; C07C63/331; C07F3/02; C07F5/00; C07F5/06

Documentation searched other than minimum documentation to the extent that such documents are included in the fields searched

    Published examined utility model applications of Japan 1922-1996
    Published unexamined utility model applications of Japan 1971-2023
    Registered utility model specifications of Japan 1996-2023
    Published registered utility model applications of Japan 1994-2023

Electronic data base consulted during the international search (name of data base and, where practicable, search terms used)

    CAplus (STN)

**C.    DOCUMENTS CONSIDERED TO BE RELEVANT**

| Category* | Citation of document, with indication, where appropriate, of the relevant passages | Relevant to claim No. |
| --- | --- | --- |
| A | LIU, Xinlei et al. Chem. Rev. 2020, 120, pp. 8303-8377<br>    pp. 8303-8377 | 1-5 |
| A | FURUKAWA, Hiroyasu et al. Journal of the American Chemical Society. 2014, 136, pp. 4369-4381<br>    pp. 4369-4381 | 1-5 |
| A | JP 2018-500157 A (NUMAT TECHNOLOGIES, INC) 11 January 2018 (2018-01-11)<br>    claims, examples, etc. | 1-5 |
| A | JP 2019-116463 A (TOYOTA MOTOR CORP) 18 July 2019 (2019-07-18)<br>    claims, examples, etc. | 1-5 |
| A | JP 2017-512637 A (THE REGENTS OF THE UNIVERSITY OF CALIFORNIA) 25 May 2017 (2017-05-25)<br>    claims, examples, etc. | 1-5 |

☐ Further documents are listed in the continuation of Box C.      ☑ See patent family annex.

| | |
| --- | --- |
| *   Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **29 May 2023** | **06 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

EP 4 495 099 A1

## INTERNATIONAL SEARCH REPORT
### Information on patent family members

International application No.

**PCT/JP2023/010265**

| Patent document cited in search report | | | Publication date (day/month/year) | Patent family member(s) | | | Publication date (day/month/year) |
|---|---|---|---|---|---|---|---|
| JP | 2018-500157 | A | 11 January 2018 | US | 2016/0160348 | A1 | |
| | | | | claims, examples | | | |
| | | | | WO | 2016/090048 | A1 | |
| | | | | TW | 201630658 | A | |
| | | | | CN | 107107028 | A | |
| | | | | KR | 10-2017-0091655 | A | |
| JP | 2019-116463 | A | 18 July 2019 | US | 2019/0194232 | A1 | |
| | | | | claims, examples | | | |
| | | | | CN | 109970778 | A | |
| JP | 2017-512637 | A | 25 May 2017 | US | 2017/0081346 | A1 | |
| | | | | claims, examples | | | |
| | | | | WO | 2015/142944 | A2 | |
| | | | | KR | 10-2016-0134644 | A | |
| | | | | CN | 107148422 | A | |

Form PCT/ISA/210 (patent family annex) (January 2015)

**EP 4 495 099 A1**

**Patent documents cited in the description**

- JP 2020176101 A
- JP 2022042378 A